# EUROPEAN PATENT APPLICATION

(11) **EP 2 742 910 A1**
(43) Date of publication of application: **18.06.2014**
(21) Application number: 14000200.7
(22) Date of filing: 24.09.2009
(51) Int. Cl.: A61C 19/00, A61C 1/08, A61L 2/00, H04N 7/18, A61B 1/247, A61B 1/05, H04N 5/225

(54) **Imaging device for dental instruments**

(30) Priority: 24.09.2008 US 99903 P
(62) Divisional of application: 09815519.5
(71) Applicant: Dentsply International Inc., York, Pennsylvania 17405-0872 (US)
(72) Inventor: Karazivan, Naim, Repentigny Quebec J5Y 3V7 (CA); Ertl, Thomas, 63691 Ranstadt-Dauernheim (DE); Guaragno, Kenneth, R., Spring Grove PA 17362 (US); Novak, Gene, Deerfield, Illinois 60015 (US)
(74) Representative: Reinhardt, Harry

(57) **Abstract**

It is an object of the present invention to provide a dental instrument comprising a drill head (52) connected to a handle, wherein the drill head has an underside and is configured to be inserted into a patient's mouth. A dental bur (20) extends from the underside of the drill head and has a long axis. An imaging device (25, 55) comprising optical components for capturing and processing an image of a drilling treatment area is incorporated in the drill head and has an optical window (53, 82) directly adjacent to the dental bur on the underside of the drill head to minimize an angle between an optical line-of-sight of the imaging device and said long axis of the bur. It has also been discovered that incorporating all optical components in the dental instrument allows for greater flexibility and manoeuvring of an imaging device.

## Description

### FIELD OF THE INVENTION

The present invention relates to an apparatus and method in the field of dentistry and in particular to optical tools for diagnosis and imaging.

### BACKGROUND OF THE INVENTION

The use of handheld dental instruments such as drills and scalers are impeded by the fact that the area to be treated can be hard to reach and that the instrument itself can block the optimal field of view of the area to be treated. Although mirrors are routinely used by dental professionals to circumvent this situation, they are both a nuisance by using up the free hand of the dental professional and hard to interpret due to image reversal. Nevertheless, it is still common for dental professionals to use handheld mirrors to visualize certain areas of a patient's mouth. It is known in the art to provide dental professionals with a camera for intra-oral use to eliminate the need for handheld mirrors.

Oral cavity cameras are used in the field of dentistry to allow dental professionals to store images of patient's mouth and to follow disease progression. Oral cavity cameras also allow the dental professional to show patients hard to view areas of their mouth before, during and after treatment. Furthermore, spectroscopic imaging cameras are used for tooth color determination allowing for proper color matching of implants. Additionally, 3-D camera-scanners are used to generate digital images of mouth architecture to facilitate computer-assisted prosthesis fabrication.

It is also known in the art to provide the dental professional with common handheld dental instruments integrating oral cavity cameras to view the patient's mouth in real-time and to concomitantly perform dental work. For example, U.S. Pat. Nos. 5,634,790, 5,052,924, 5,049,070 all disclose dental instruments (drills) incorporating cameras which allow the dental professional to benefit from enhanced viewing of the operative field in real-time. These three objects of the prior art all pertain to imaging devices using fibre-optics to relay the image to a sensor located inside the handpiece of the dental instrument. Furthermore, in all three patents, the dental instrument must be designed and/or significantly modified to allow for incorporation of a camera.

In U.S. Pat. Pub. No. 2008/0090199, Noguchi teaches an apparatus combining a visual camera with optical coherence tomography (OCT) to view and diagnose certain dental pathologies. In the device, OCT is combined with an optical camera in order to provide an apparatus which both depicts images of the surface of patient's teeth and gums as well as an inner picture of these tissues. Noguchi also teaches an imaging device where the image sensing component is in the distal end of the device. It is important to note that the device of Noguchi is exclusively an imaging device and does not teach a method to add imaging capability to a standard dental instrument.

In U.S. Pat. No 5,328,365 Jacoby teaches an instrument for endoscopic subgingival identification of dental tartar while concomitantly removing the tartar using the same instrument. Like most endoscopic devices, Jacoby's apparatus contains imaging sensing capability located outside the endoscopic device at the distal end of the fibre optic bundle. Most endoscopic devices are designed in this way because cameras are larger than the anatomical passageway into which they are inserted. Furthermore, in a typical endoscopic device where the image sensing capability is removed from the image capture capability, a portion of the reflected light and resolution is lost due its passage through a fibre optic bundle.

### SUMMARY OF THE INVENTION

It has been discovered that dental professionals can benefit from adding image viewing capability to several standard dental instruments such as a drill, a curette, a scaler, an extractor, a surgical instrument, a mirror and a toothbrush. A viewing device is provided where all camera components such as illumination, image capture and image processing are integrated into a structure allowing easy attachment and detachment from the tip of a dental instrument to enhance viewing of the operative field. Easy attachment and detachment of an imaging device can also allow the removal of an imaging device prior to sterilization of a dental instrument.

It has also been discovered that incorporating all optical components in the distal tip of a dental instrument allows for greater flexibility and manoeuvring of an imaging device due to the absence of a bulky fibre optic bundle between image capture, processing and display.

It has also been discovered that, when an intra oral camera is combined with a dental drill, the angle between the camera's line-of-sight (i.e. optical axis) and the drill bur axis needs to be as small as possible in order to maintain a proper view of the "drilling" area as the end of the drill enters into the tooth.

The invention uses small imaging chips. In addition to removing the need for fibre optics, using a miniaturized camera has the added benefits of reducing the size of the viewing area of the assembly hence allowing it to conveniently fit on a dental handpiece without compromising the visual field and physical access to the field, thus also allowing to define a low angle between the longitudinal axis of the instrument and the working tip, utilising very small electric conductors to attach the camera to the rest of the electronic compartment, obtaining a good depth (few mm) of the field of view and a small focussing distance (few mm) between the imaging sensor and the field to image and finally lowering the cost of the device when compared to more complex optics.

It is an object of the present invention to provide a device and method which allows dental professionals to visualize in real-time the inside of a patient's mouth without the need for mirrors. It is a further object of the present invention to design a system and method allowing for easy attachment and detachment of a camera to standard dental instruments. In the present invention, a camera where all required optical components (lens, sensor, illumination) are encapsulated for sterilization purposes (autoclaving) and attached to the tip region of an instrument. Furthermore, coarse camera adjustments can be made by moving the camera along the tip of the dental instrument and fine adjustments can be performed by mounting, for example, the encapsulated camera on a ball joint.

It is another object of the present invention to provide a device for adding imaging capability to a dental instrument comprising an adapter with a chamber adapted for receiving and removing an imaging device, the adapter secured to a dental instrument to isolate the imaging device from the dental instrument in said chamber such that a non-sterile imaging device can be used with a sterile dental instrument without compromising sterility of the dental instrument and a removable imaging device wherein electronic image detection occurs at the instrument head.

It is an object of the present invention to provide a dental instrument with optical means to localize specific hard to view/reach areas of the mouth, after which the optical means can be removed through a quick release mechanism and the dental work such as drilling or scaling can be performed without optical means.

It is object of the present invention to provide an optical device that is attached to the instrument or embedded into the instrument that contains all optical components for illumination, image capture, image processing and image transmission at the working end of a dental instrument. When transmitting an image away from the working end of a dental instrument, having all optical components at the working end allows for transmission of an electrical signal to an image viewer as opposed to an optical signal when a fibre optic bundle is used. On one hand, electric wires are more convenient and flexible that fibre optic bundles and on the other hand, capturing an image directly at the working end of the dental instrument allows for greater resolution and decreased loss of signal.

It is another object of the present invention to provide an optical device which is easily attachable to the working end of a dental instrument to perform dental work and easily detachable thereafter. In order to fit an optical device on the working end of a dental instrument, it must be designed in such a way that the optical device is small enough not to inhibit normal use of the dental instrument.

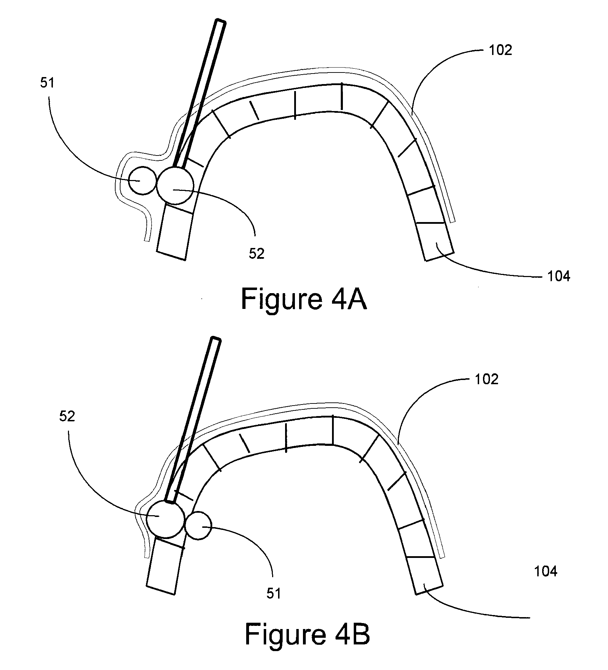

It is also an object of the present invention to provide a system which allows the position and angle of the optical device to be adjusted on the working end of a dental instrument and this can be achieved by a magnet or clip attachment or any other means which allows easy attachment and detachment. It will become clear from the description (Fig.4) why this is an important consideration in the case of an attached optical device.

It is yet another object of the present invention to provide a chip that generates a video signal directly inside a casing in order to reduce noise on the video signal and therefore enable electrical wiring to relay image data without loss or damage of signal due to electromagnetic field generated by the dental instrument.

In other embodiments, the optical device can be attached to dental instruments such as those used for extractions, soft tissue restorative surgery instruments, periodontics instruments such as scalers and curettes, diagnosis instruments to record location of the area to monitor or to treat, intra-oral cameras with physical reference support to keep a good focus and to facilitate handling and 3D imaging acquisition devices while using the instrument.

Additionally, attaching an imaging device onto many dental instruments allows dental professionals to work on or treat patients without physically being in the office, allows dental professionals to show their patients the treatment being performed as well as before-after images of the work area, allows for occlusion verification tool from lingual aspects, allows to monitor for temporal disorders inside an occlusal plate.

In yet other embodiments, there is provided a dental drill comprising an intraoral camera adapted to provide images of a drill site, the intraoral camera comprising an image sensor integrated circuit chip having an optoelectronic area and image processing area, the optoelectronic image acquisition area being offset from a center of the chip, the chip being arranged at a distal end of the drill with offset the optoelectronic image acquisition area closer to a drill bur axis, wherein an angle between the drill bur axis and an optical axis of the camera is reduced by the offset.

In still other embodiment of the present invention, there is provided a dental handpiece comprising an optoelectronic device for intraoral optical diagnostics and/or imaging, a socket located at a distal end for removably receiving and connecting the device, an electrical cable connected between the socket and a proximal end of the handpiece.

In some aspects of the present invention, there is provided a method of protecting an optical window or camera using hydrophilic material to establish a liquid layer thus enhancing the quality of images captured.

In other aspects of the present invention, there is provided a dental handpiece comprising an imaging device mounted with tongue/cheek retractors to observe a work area inside the mouth that is otherwise blocked by tongue and cheeks or by tongue and cheek retractors.

In yet other aspects of the present invention, there is provided an imaging device with a thin wall close to the viewing window of the imaging device to enable/facilitate evacuation of water. This is especially useful when a dental drill bur is rotating at high speed wherein the generated vortex increases the accumulation water at the viewing window.

In yet other applications of the imaging device, it can be used instead of a microscope if sufficient zooming power is available, it can be used to isolate field devices (e.g. Isolite®), it can be attached to a finger of the operator when using endodontic files, it can be used to obtain endoscopic stereoscopic vision, it can be used to obtain pocket measurements by focussing on the probe with color coded depth.

### BRIEF DESCRIPTION OF THE DRAWINGS

FIG.1 is a schematic side view of an imaging device attached to a dental instrument.
FIG.2 illustrates an encapsulated imaging device relative to the distal end of a dental instrument such as a drill.
FIG.3 illustrates a magnified view of the physical arrangement of optoelectronic components of an imaging device capsule.
FIG.4 illustrates a dental instrument with a camera performing work inside the mouth to highlight usefulness of an adjustable camera.
FIG.5 illustrates one embodiment of the invention where an imaging device is easily attachable and adjustable on the working head of a dental instrument such as a drill.
FIG. 6 illustrates one embodiment of the invention where the image capture, processing, transmission, control, and viewing components are depicted.
FIG. 7 pictures the imaging device with standard dental instruments.
FIG. 8 is a schematic side view of an assembled preferred embodiment of the present invention.
FIG. 9 illustrates an exploded side view of the various components of a preferred embodiment.
FIG. 10 is a schematic representation of another embodiment of the optoelectronic components of an imaging device.

### DETAILED DESCRIPTION OF THE EMBODIMENTS

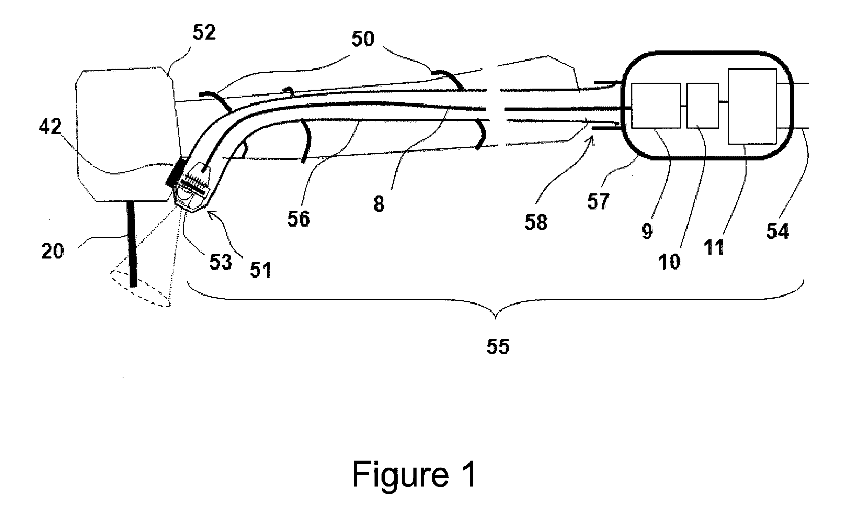

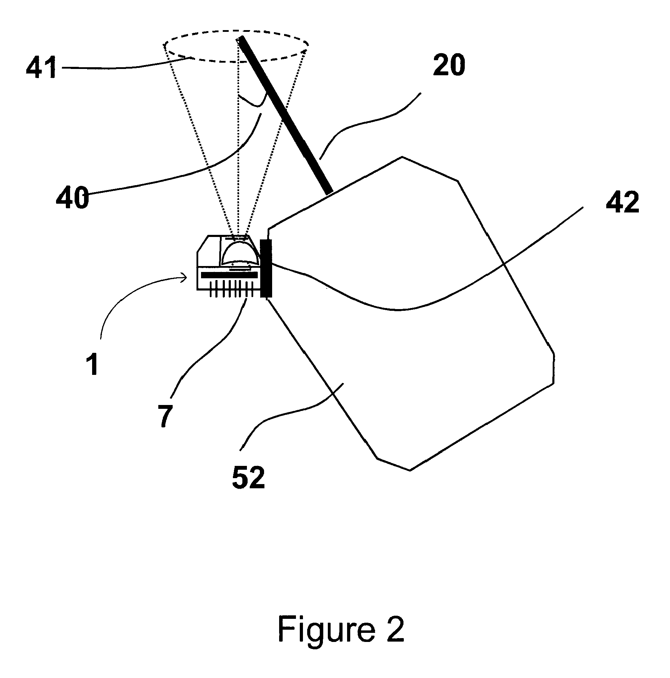

Referring to the drawings and for illustrative purposes, the present invention can be embodied by the device shown in FIG.1 which illustrates the attachments **50** of the imaging device **55** to a dental instrument such as a drill **52** with a working tip such as a bur **20.** Attachment of the imaging device **55** to the dental instrument **52** can be achieved by a mounting device **42** composed of an attachment body casing made of materials such as silicon or plastic and comprising a means to easily attach, position and detach the imaging device on a dental instrument. The electrical wires **8** allowing transmission between the optical device head **51** and control unit 57 can be secured to the dental instrument **52** with an easy clipping mechanism **50** whereas the optical device head **51** can be secured to the dental instrument head **52** by a permanent magnet found on the mounting device **42.** Magnetic mounting of the device head to the instrument head is advantageous as it allows for optimal positioning of the device head **51** on the dental instrument head **52.** The device head **51** contains at least one optical window **53** which allows for both illumination and image capture as well as all the necessary encapsulated miniaturized optical components described in more detail in FIG.2. Keeping the outside of viewing window **53** clean can be achieved by a standard air-water spray, by manually cleaning it with a cloth or by using permanent or temporary anti-fogging material such as oil or grease or a hydrophilic agent to create a layer of liquid, thus preventing fog. The inside of the tube can be cleaned, as required, using small brush and/or compressed air.

In the embodiment of FIG.1, the window **53** is a flat window. However, in other embodiments, the window can be a lens that provides optical power for imaging.

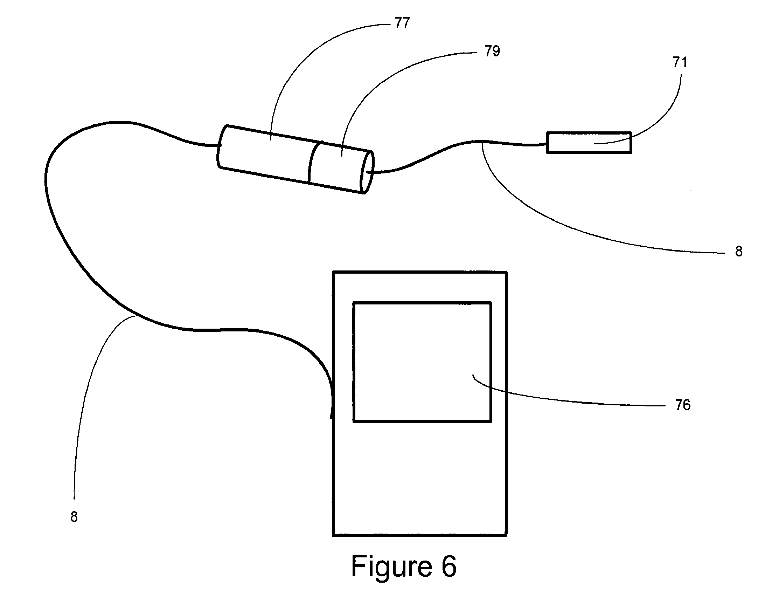

The image sensor assembly **2** has protruding connectors **7** which allow it to be connected to the distal casing **57** through electrical wires **8** inside a flexible supporting tube **56** to protect the wires **8** and help guide the imaging device into the tube. This protective tubing can be made of insulating material such as that used for coaxial wires. The proximal casing **57** contains electromagnetic interference shielding, an electronic module for imaging and illumination control such as controllers for LED (light-emitting diode) intensity, exposure, aperture correction, gamma correction, gain control, white balance, color saturation, zoom function, image rotation function as well as other electronic controls **9** and a wireless RF transmitter **10** to send the image wirelessly to an external viewer or recorder. Alternatively, the image data can be sent to a viewer through an electrical wire (FIG.6) or infra-red data transmission. Image rotation is an important functional aspect of the device and several methods can be used to achieve image rotation such as directly adjusting camera position, manually turning the viewer or by electronic processing. The electronic processing can be used to automatically rotate the image axis both horizontally and vertically depending on the location of the instrument inside the mouth. Orientation of the dental instrument can be determined automatically by an accelerometer for example.

The proximal end of the imaging device is adapted to provide a power source **54** such as that from external alternative current or internal power from batteries **11** or induction means. In the present embodiment, a connector **58** is provided which allows easy connection and disconnection of the distal from proximal ends of the device so as to permit sterilization of the dental instrument and distal end of the imaging device either by autoclaving or chemical sterilization such as ozone or ethylene without having to sterilize sensitive electronic equipment found in the casing **57.**

In the embodiment illustrated in FIG.1, the dental handpiece has a tube that is removable from the handpiece for receiving the camera. The tube includes an optical window at its end. The camera can be separated from the tube to allow sterilization of the tube and the dental handpiece without ever needing to sterilize the camera. Inserting of the camera into the tube and the sealing at connector **58** eliminates any exposure or transfer of pathogens between the camera and the oral cavity environment to protect the health of the patient.

The tube may be removable from the handpiece as shown, or it can be integrated into the handpiece in other embodiments. The tube and its end window can be made from materials that can withstand autoclaving.

In some embodiments, the image sensor chip, lens and illumination LED package is removably connected in a socket at the distal end of the handpiece. The socket can provide an electrical connection between the camera and the proximal controller and/or display unit and/or casing 57. The socket can allow the camera to be removed, if desired, before sterilizing the handpiece. For example the camera can be chemically sterilized and the handpiece autoclaved. The socket can also allow the camera unit to be easily replaced if it fails. The distal camera unit comprising the image sensor chip, LED and imaging lens can be manufactured at low cost.

Image data can be recorded directly in a memory storage device found inside the electronic casing **57.** Alternatively, image data can be sent to a computer found in the dentist's office. An optical button can be conveniently added to wires **8** consisting of an emitter and receiver for detecting disturbances in light transmission. This optical button could have multiple functions such as adjusting illumination intensity; acquire image data and controlling optical parameters.

The proximal casing **57** can be mounted to the proximal end of the handpiece. In the embodiment of Figure 6, the display screen is separate from the handpiece. In some embodiments, the casing **57** comprises a liquid crystal display screen. In some of such embodiments, the casing **57** is mounted to the handpiece in a rotatable manner, for example rotatable about the proximal handle of the dental drill. Thus if a drill is being used in different orientations, namely with the bur up or down, the screen can be rotated to face the dentist. It will be appreciated that in embodiments in which a tube is removably added to the handpiece, the tube can fit between the thumb or index finger and the handle when the user grips the instrument.

In embodiments where the display screen is mounted on the proximal end of the handle of the dental instrument, the screen can be located proximal of the user's grip on the handle and be in a good position for viewing without interfering with the use of the instrument.

FIG.2 illustrates the miniature encapsulated imaging sensor assembly **2** relative to the distal end of a dental instrument **52** and the working tip **20** of a rotating bur. Adjustments can be made to optimally position the image sensor assembly **3** in such a way as to minimize the angle **40** (FIG.2) between the long axes of the working tip bur and the line-of-sight of the camera intersecting the distal end of the working tip bur in a focal plane defined by **41.** A lens can be adjusted so as to focus substantially at the end of the working end drill bur **41.**

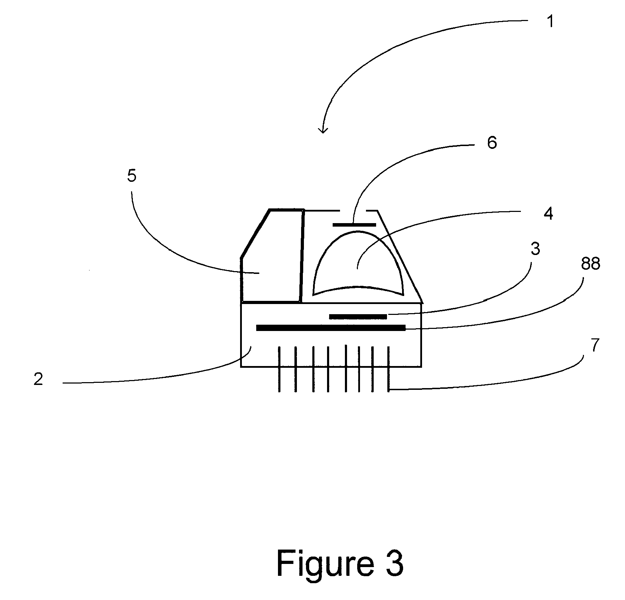

FIG.3 illustrates the distal end of the miniature encapsulated imaging device **1** where an asymmetrical housing contains all optical components such as an imaging sensor assembly **2,** namely an Omni-Vision OV6920 which has a very small footprint (2mm x 2mm x 1mm), the imaging area of the image sensor **3** which is a CMOS-type sensor but can also be a CCD-type sensor located on a chip/printed circuit board (PCB) **88,** a focusing lens **4** which can be made of glass or plastic and adjusted by an adjusting the position of the lens or alternatively by altering the position of the imaging device on the dental instrument.

The Omni-Vision OV6920 integrated circuit has its image sensor region 3 in one corner of the chip surface with the rest of the chip serving image processing or transmission functions **88.** The offset image sensor region is used to place the optical axis closer to one side of the encapsulated assembly so that the angle **40** can be reduced. The offset image sensor also makes it possible to locate small illumination LEDs over the chip in the non-image sensor area so that the illumination source and the imaging optics can be packaged as close as possible to one another and decreasing total footprint of the imaging device.

The optical components also comprise a light emitting diode (LED) area **5** which can contain one or more LEDs of one or more wavelengths such as white, ultra-violet, infra-red, laser or other for illuminating or diagnosing the work area, an infra-red filter **6** or any other filter to prevent entry of unwanted wavelengths, and wires to electrically connect an image sensor to an image viewer and/or recorder. The light source **5** can illuminate a different field than that detected by an optical window to prevent specular reflection when a prism or mirror is used to divert an image to the detector. The imaging device 1 capsule can be made of aluminum or any other metal or heat resistant plastic and can be epoxy-filled to resist temperatures associated with standard autoclaving or chemical sterilization by ozone or ethylene. Furthermore, the capsule should be air tight and impermeable to liquids. Stereoscopic images can be acquired by placing another imaging device on the dental instrument.

FIG.4 sketches a dental instrument with a camera performing work inside the mouth of a patient to highlight one of the advantages of having an imaging device with adjustable positioning with respect to the dental instrument. It can be appreciated that the camera position on the dental instrument in FIG.4B would be more comfortable for the patient than that of 4A due its optimal localisation thereby limiting skin extension. Furthermore, if dental work is performed in the back of the mouth, it is not ideal to have the camera positioned at the top of the dental instrument for accessibility reasons.

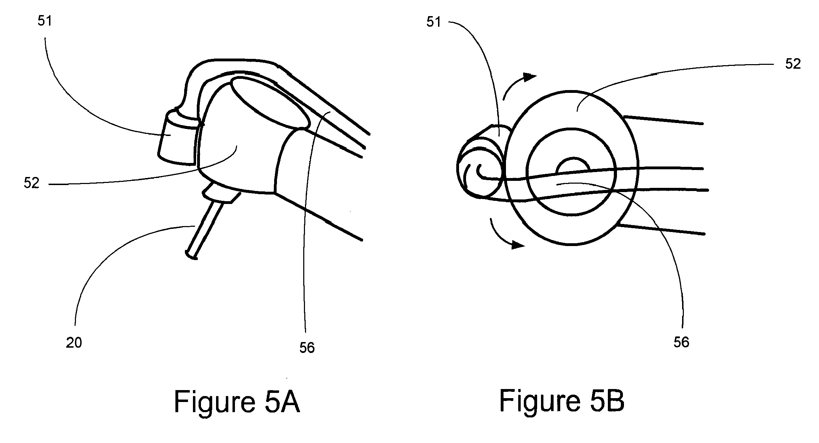

FIG.5 pictures one embodiment of the invention where an encapsulated imaging device **51** is attached to a dental instrument head **52,** in this case a drill with a drill bur **20.** It can be appreciated from the image that the encapsulated optical device can be displaced along the circumference of the instrument head as can be seen by the arrows in the top view. Image quality and patient comfort are two reasons why it is important to optimally place the imaging device on the dental instrument head. Indeed, it is an object of the present invention to provide an imaging device where all optical components can be found at the working end of a dental instrument, hence putting the image sensor in direct path of the image and bypassing the need to transmit the image through a fibre optic bundle.

While not shown in Figure 5, in some embodiments, the drill can be provided with a flexible shroud that prevents a patient's cheek or tongue from coming into contact with the bur. By providing an intra-oral camera within the field of operation, the shroud can be used without concern as to its impact on seeing the field of operation since the camera image is used to guide the dentist.

The attachment of the distal camera unit to the handpiece distal end can also be done using an adapter that mates or clips securely to the distal end of the handpiece. Several notches into which the imaging device can be placed along the circumference of the dental instrument head can allow for more secure fastening of the imaging device to the dental instrument head **52.**

FIG.6 pictures the imaging components **71** such as the optical device head containing the image capture and processing components which is connected to the Image control components **79** through electrical wires **8.**

These image control components **79** can be found either inside the handpiece if the device is embedded into a dental instrument or conveniently attached to the handpiece if the device is in the attachable format. The data is then transferred through an electrical wire in real-time to the image viewer **76.** The device shown in FIG.6 is battery powered through battery **77.**

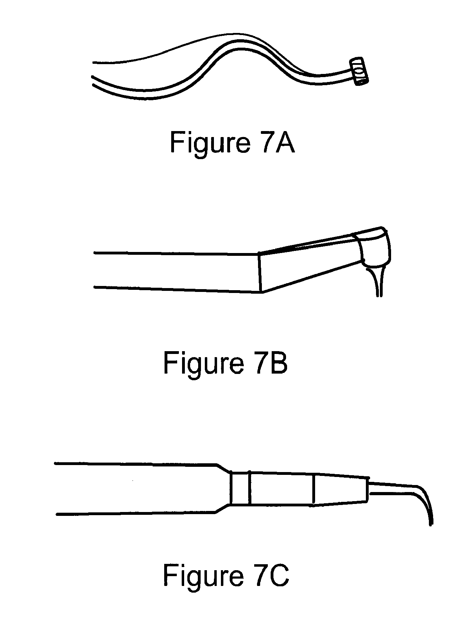

FIG.7A shows the imaging device with respect to two standard dental instruments such as a dental drill (FIG.7B) and a scaler (FIG.7C) to show the relative size of each and to highlight the multiple uses of an adaptable imaging device.

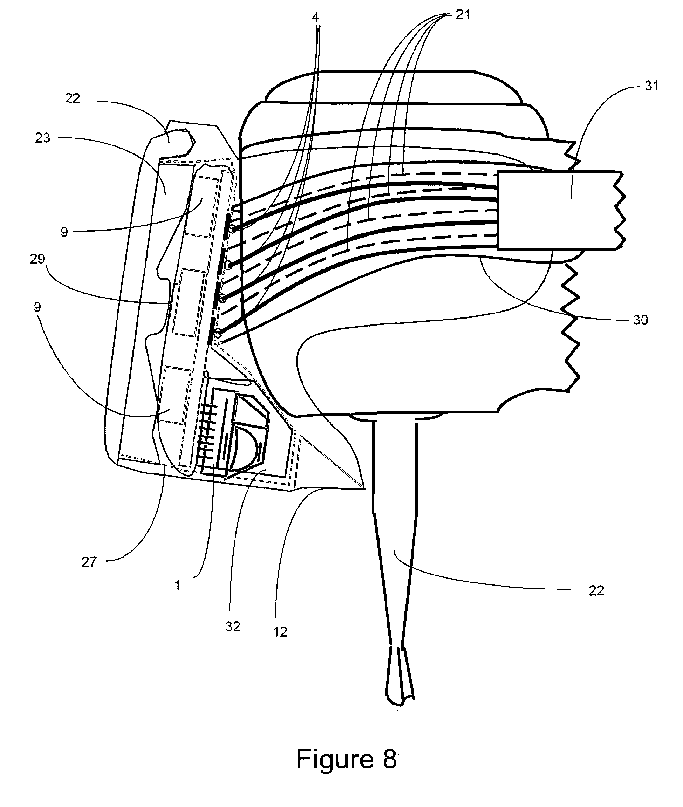

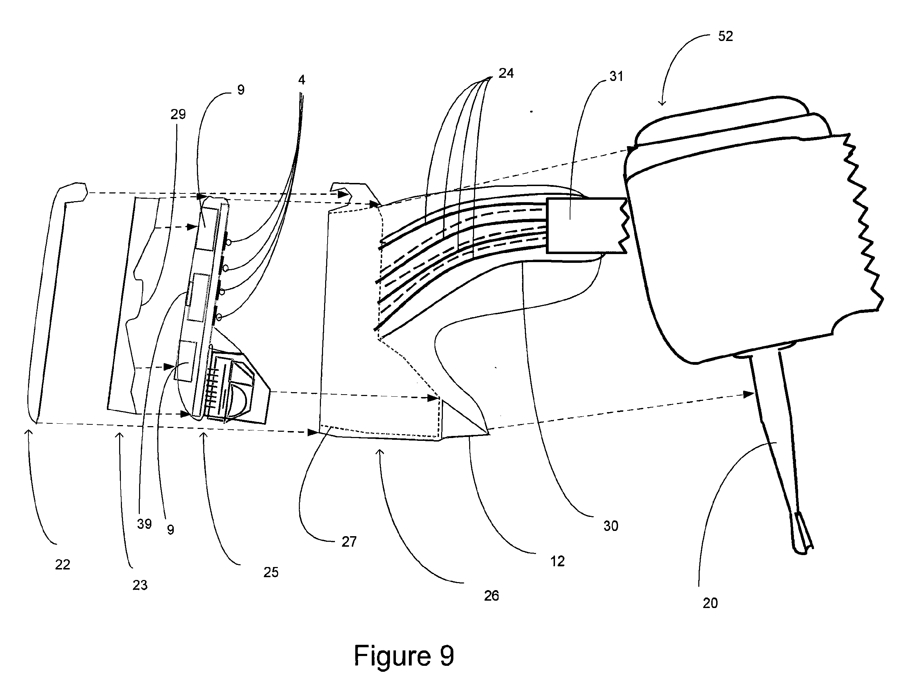

FIG.8 shows a completely assembled side view image of one preferred embodiment of the invention while FIG.9 shows the same embodiment separated/exploded into its component parts. This embodiment comprises a drill head adapter **26** which can be secured to a drill head **52,** and comprises a chamber **27** for receiving a removable imaging device **25** that can be conveniently inserted and removed, using a snap-in mechanism for example, into the drill head adapter **26.** A snug piece **23** which can be made of a material such as silicone and used to insure impermeability and a snug fit and proper electrical contact between the imaging device **25** and the drill head adapter **26** through connection mechanism **4** and **21.** The cover **22** and snug piece **23** can be combined into one single cover piece. The cover piece **22** allows for non-sterilized removable imaging device **25** to be combined with a dental drill without compromising sterility of the dental drill. The snug piece **23** and cover **22** can be used to seal off the imaging device **25** in the drill head adapter, rendering it impermeable to aqueous contaminants such as water saliva and blood but more importantly allowing the dental instrument to be sterile on the outside despite non-sterile components inside the chamber **27** of the drill head adapter **26.** The snug piece **23** must be rigid enough to allow pressure on cover **22** to actuate, through protruding section **29,** a button **39** (such as an on/off or image adjustment buttons) of the image device **25.** Snug piece **23** can also be attached to the removable imaging device through a hinge in order to decrease the number of independent components. The securing mechanism **31** allows the drill head adapter to be securely fastened to the drill and can act as a passage and/or connector for the wiring **8.**

This type of assembly allows sterilization of all components of the dental drill imaging device that can come in contact with a patient including the cover **22** which is snapped back into place (without the imaging device **25)** prior to sterilization. In this embodiment, electronic components **9** can be combined to the imaging device **25** in order to minimize the number of wires exiting the drill head adapter. It will be appreciated by those skilled in the art that this drill head adapter for adding imaging capability can be adapted to any other dental instrument such as a curette, a scaler, a mirror, a toothbrush, a surgical instrument and an extractor.

A battery and wireless transmitter can be placed inside the drill head adapter (or the removable imaging device **25**) in order to eliminate the need for wires. However, if wires are used such as in FIG.8, the wires can enter drill head adapter to provide, in addition to external image control, the required electric current for function. As seen in FIG.8, the drill head adapter is fitted onto the drill head and comprises a mirror 12. The drill head adapter and mirror can be permanently secured to the dental drill with autoclave resistant glue such as epoxy. All chemical and/or heat sensitive components can be included in the removable imaging device **25** to avoid damage caused by sterilization.

As can be seen in FIGS.8 and 9, the drill head adapter comprises but does not cover the mirror **12.** The mirror **12** allows for the angle between the drill bur **20** and the line-of-sight of the camera to be minimized. It will be appreciated by those skilled in the art that when the angle **40** is too wide and the tip of the drill penetrates inside the surface of the tooth, the end of the drill bur is no longer in the line-of-sight of the camera (i.e. visible), which would then capture the tooth surface rather than the drilling part of the drill bur. Alternatively, the imaging device **1** can be placed directly adjacent to the drill bur however this would be more cumbersome than a small mirror. The removable imaging device comprises all components shown outside the dotted line (i.e. the chamber **27** of the drill head adapter **26**) with the exception of the cover **22.**

The imaging capability 22-26, is positioned at the distal end of the dental handpiece for space optimization purposes and to facilitate use of the dental instrument. The removable imaging device **25** makes electrical contacts with the drill head adapter **26** through electrically conducting/connecting elements **4** and the wires **24** which are part of the drill head adapter **26.** The electrically conducting elements can be made of any conducting material such as copper or stainless steel. In order to reduce interference and prevent short circuits between the wires 24, separation grooves/walls 21 can be provided.

The part of the sheath for receiving the removable imaging device surrounding the drill head adapter which faces the drill bur can be made of a thin and/or translucent material such that light emitting diodes inside the removable imaging device **25** (which can be made of the same material) can illuminate the work field without specular reflection in the mirror. It will be appreciated that the material must be autoclavable because it is part of the drill head adapter which undergoes sterilization with the dental instrument.

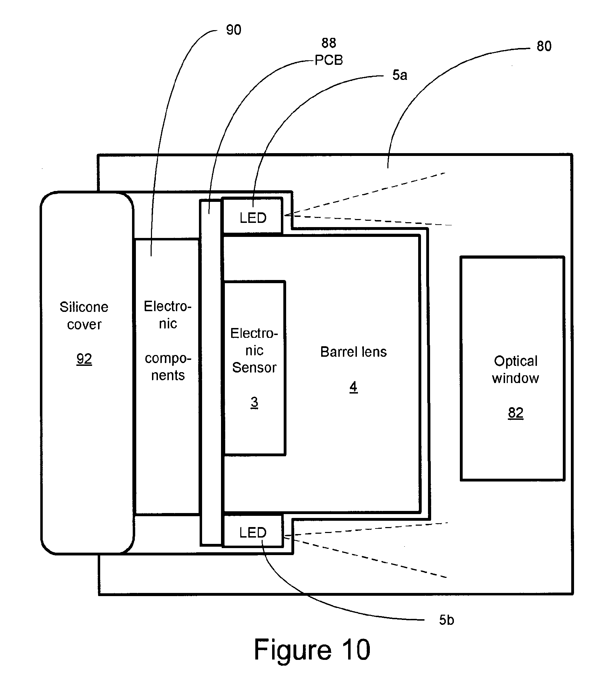

FIG. 10 is a schematic representation of alternative embodiment of the optoelectronic components of an imaging device **80** as shown in FIG.3. In this embodiment, a barrel lens **4** is placed in the middle of the chip/PCB **88** thus allowing for LEDs **5a** and **5b** to be placed on both sides of the lens **4** and detector **3.** This allows to better control of illumination intensity and waveband such that multiple illumination colors can be used. Electronic components **90** are sandwiched between the PCB **88** and the silicone cover **92.**

Imaging capabilities on a dental instrument can find numerous applications in the dental field. For example, miniature imaging capabilities on a toothbrush can be exploited for teaching purposes, especially for children learning how to brush their teeth. Also, imaging capability can be added to articulation paper used to verify occlusion (bite) or cracked teeth.

Furthermore, having viewing capabilities on a dental drill can help in the determination of drilling quantity, quality and to evaluate cavity preparation. Indeed, when a dental professional drills a tooth to remove carious material, knowledge about when to stop drilling can be obtained from both feeling the drill bur on the tooth but also by observing the visual characteristics of the drilled/shredded material projecting from the drilling site.

Imaging capability on a standard mirror can provide a useful way for a dental professional to examine the mouth of a patient, whilst recording visual images of the patient's mouth. These images can be used for reconstruction of a 3D images of the patient's mouth and teeth. Indeed, images from the dental professional's exploration can be stitched together and used to evaluate treatment quality and/or progress over time.

An imaging device in place of a mirror can be provided including a small screen outside the mouth on the back end of the mirror handle. A standard mirror can be on the opposite side of the imaging device. This solves the problem or at least slows the process of water droplets disturbing the camera image, as typically happens with a mirror. A hydrophilic material can be provided to protect the imaging device and to enhance the quality of images captured.

By using an appropriate detector/CCD, the imaging device can be used in the infrared and ultra-violet spectra in order to detect or capture images where IR and UV can be of informational and/or diagnostic value. Specific LEDs can be used for pulsing in specific wavebands in order to obtain differential images indicative of diseases such as caries and periodontal disease. In fact by having a wide waveband detector and either waveband specific LEDs or filters, the imaging device can act as a miniature spectrophotometer. The imaging device can be used to illuminate specific points with structured light in order to evaluate the amount of light adjacent to an illumination point.

The invention may comprise the following additional preferred aspects:
1. An intraoral camera comprising:
   an image sensor and at least one illumination light source encapsulated together;
   a removable mounting for attaching the encapsulated image sensor and light source to an external surface of a distal end of a dental instrument to illuminate and image a working tip of a dental instrument;
   a proximal unit for delivering power to said image sensor and said light source, and for processing, transmitting and/or displaying images from said image sensor;
   a cable connected between said encapsulated image sensor and light source and said proximal unit; and
   a relaying device for relaying images from a proximal unit to an image viewer.
2. The intraoral camera as mentioned in aspect 1 wherein said encapsulation is adapted to protect said intraoral camera during sterilization.
3. The intraoral camera as mentioned in aspect 1 or 2 wherein said intraoral camera is adapted to minimize the angle between an optical axis and a drill burr axis.
4. The intraoral camera as mentioned in any one of aspect 1 to 3 wherein said attaching is performed by one of a magnetic, a clipping and a notch mechanism.
5. The intraoral camera as mentioned in any one of aspect 1 to 4 wherein a protective single-use barrier is placed on said imaging device to maintain sterility of said dental instrument.
6. The intraoral camera as mentioned in any one of aspect 1 to 5 wherein said relaying device is one of a coaxial cable and an radiofrequency transmitter.
7. The intraoral camera as mentioned in any one of aspect 1 to 6 wherein the image viewer is one of a computer screen, glasses, an external screen and a retractable device that attaches to the glasses or head of a dental professional.
8. The intraoral camera as mentioned in any one of aspect 1 to 7 wherein said illumination is provided by a light emitting diode and can illuminate in multiple wavebands.
9. A method of sterilizing a dental instrument with imaging capability comprising:
   removing said imaging capability from a chamber attached to but microbiologically isolated from said dental instrument;
   sterilizing said dental instrument including said chamber; and
   reinserting said imaging capability into said chamber without contamination of said dental instrument to prepare said dental instrument for dental work.
10. A method of viewing an intra-oral space while working with a standard dental instrument comprising:
   attaching an encapsulated miniature optical device to said dental instrument;
   positioning said encapsulated miniature camera at the distal tip of said dental instrument
   adjusting said encapsulated miniature camera in such a way as to minimize the angle with the long axis of instrument
11. A method of viewing an intra-oral space while working with a standard dental instrument further comprising using an imaging device according to any one of aspect 1-24.

It will be appreciated by those skilled in the art that image stabilisation techniques and components could be useful when imaging capability is added to a dental drill with high vibration. Furthermore, although image focussing techniques and image processing techniques are known in the art, they can be applied advantageously within the present intraoral camera for added functionality.

## Claims

1. A dental instrument comprising:
a drill head (52) connected to a handle said drill head having an underside and configured to be inserted into a patient's mouth;
a dental bur (20) extending from the underside of the drill head and having a long axis, and
an imaging device (25, 55) comprising optical components for capturing and processing an image of a drilling treatment area, the imaging device incorporated in the drill head and having an optical window (53, 82) directly adjacent to said dental bur on the underside of the drill head to minimize an angle between an optical line-of-sight of the imaging device and said long axis of the bur.

2. The dental instrument of claim 1, wherein the imaging device (25, 55) is adjustably connected to the drill head.

3. The dental instrument of claim 1 or 2 wherein the imaging device (25, 55) is electrically coupled to an image viewer.

4. The dental instrument of any one of claims 1 to 3 wherein the imaging device further comprises a light source (5) provided by light-emitting diodes (5a), the light-emitting diodes (5a) able to emit at various wavelengths.

5. The dental instrument of any one of claims 1 to 4 wherein the imaging device (25,55) comprises an image sensor (3) being sensitive to light from the infrared spectrum.

6. The dental instrument of any one of claims 1 to 5 further comprising an image stabilisation device to compensate for vibration of said dental instrument.

7. The dental instrument of any one of claims 1 to 6 further comprising a rotatable mounting configured to be connected to the handpiece at a proximal end of a handpiece rotatable about at least one axis of the handpiece and a display screen (76) mounted to the mounting.

8. The dental instrument of any one of claims 1 to 7wherein the dental instrument (52) further comprises tongue/cheek retractors such that the imaging device can capture images of a work site that would otherwise be blocked by said tongue/cheek retractors.

9. The dental instrument of any one of claims 1 to 8 further comprising a chamber (27) secured to the drill head for receiving and removing an imaging device (25,55); the chamber (27) also adapted for isolating the imaging device (25,55) from the dental instrument (52) such that a non-sterile imaging device (25,55) can be used with a sterile dental instrument (52) without compromising sterility of the dental instrument (52); the chamber (27) comprising the optical window (53,82).

10. The dental instrument of claim 9, wherein the imaging device (25,55) comprises a printed circuit board (88), and the chamber (27) is arranged to hold the circuit board (88) on a side of the drill head with an image sensor chip (3) mounted on the circuit board (88) on the underside of the drill head.

11. The dental instrument of any one of claim 9 or 10, wherein isolating the imaging device is achieved by one of attaching a removable cover (22) and closing a hinged cover to isolate said chamber.

12. The dental instrument of any one of claims 9 to 11, wherein the chamber (27) is a tube (56) having an open proximal end into which the imaging device can be inserted and removed, and a distal end closed by the optical window (53).

13. The dental instrument of any one of claims 1 to 12 wherein an electrical connection (4,21) between an adapter (26) and the imaging device (25,55) is achieved by pressing the imaging device (25,55) into the adapter (26) such that electrically conducting elements from both components come together to form the electrical connection (4,21).
